Europäisches Patentamt

European Patent Office

Office européen des brevets

(11) Publication number: **0 333 490 B1**

(12) **EUROPEAN PATENT SPECIFICATION**

(45) Date of publication of patent specification: **18.08.93**  (51) Int. Cl.⁵: **A61K 9/50**, A61K 37/50, A61K 37/02

(21) Application number: **89302629.4**

(22) Date of filing: **17.03.89**

(54) **Composition for the treatment of hepatopathy.**

(30) Priority: **18.03.88 JP 65337/88**

(43) Date of publication of application:
**20.09.89 Bulletin 89/38**

(45) Publication of the grant of the patent:
**18.08.93 Bulletin 93/33**

(84) Designated Contracting States:
**DE ES FR GB IT**

(56) References cited:
**EP-A- 0 225 130**
**EP-A- 0 240 346**
**WO-A-87/01387**
**US-A- 4 024 247**

(73) Proprietor: **ASAHI KASEI KOGYO KABUSHIKI KAISHA**
**2-6, Dojimahama 1-chome Kita-ku**
**Osaka(JP)**

Proprietor: **Tosoh Corporation**
**4560, Kaisei-cho**
**Shinnanyo-shi, Yamaguchi-ken(JP)**

(72) Inventor: **Nakae, Dai**
**4-500-34 Higashi-Ikoma**
**Ikoma-shi Nara-ken(JP)**
Inventor: **Konishi, Yoichi**
**No. 215 Jyoroku**
**Sakai-shi Osaka-fu(JP)**
Inventor: **Nagata, Yoshihiko**
**1-9-1-203 Minamidai**
**Sagamihara-shi Kanagawa-ken(JP)**
Inventor: **Kobayashi, Youshiro**
**719-5 Moriki Ohito-cho**
**Tagata-gun Shizuoka-ken(JP)**

(74) Representative: **Woods, Geoffrey Corlett et al**
**J.A. KEMP & CO. 14 South Square Gray's Inn**
**London WC1R 5EU (GB)**

## Description

This invention relates to a therapeutic composition for the treatment of hepatopathy.

The liver is a large organ in the body and has the various activities of metabolizing carbohydrates, proteins, lipids, nucleic acids, vitamins and hormones, detoxicating and eliminating endogeneous or exogeneous substances by oxidation, reduction or conjugation, generating blood coagulation factors, regulating blood circulation and controlling sugars. It is an essential organ to maintain life as it also synthesises or produces proteins, urea and vitamin D intermediates.

The liver is easily exposed to various damaging factors such as viruses, alcohol and drugs (toxins) causing hepatopathy with symptoms such as fatigue, anorexia and jaundice. Pathological findings, for example inflammation, degeneration, necrosis or infiltration of hepatocytes, may be observed.

The main cause of hepatopathy is the hepatitis virus, next are alcohol and drugs, which cause acute viral hepatitis, acute alcoholic hepatitis and acute drug induced hepatitis respectively. Furthermore chronic hepatitis is known. For these, necrosis of hepatocytes and increase in serum GOT and GPT are observed in pathological tests and in liver function tests respectively.

The fundamental curative treatment is generally rest, dietetics and trophotherapy through drip infusion. For alcoholic hepatitis, total abstinence, and for prolonged jaundice with drug induced hepatitis, administration of adrenocortical hormone or phenobarbital, are recommended.

Recently, so called hepatic disease drugs, for example liver extract or glutathione protein synthesis stimulant, have been used. However more potent drugs with less side-effects are expected to be developed.

Among polypeptides having superoxide dismutase activity, a polypeptide containing copper and zinc (Cu, Zen-SOD), a polypeptide containing manganese (Mn-SOD) and a polypeptide containing iron (Fe-SOD) have been known to be widely distributed in animals, plant and microorganisms [Advances in Enzymology, 41: 35 - 97 (1974)]. Superoxide dismutase catalyses the reaction shown below, on a superoxide anion radical ($O_2^-$) to generate hydrogen peroxide ($H_2O_2$) and molecular oxygen.

$$2\,O_2^- + 2\,H^+ \rightarrow O_2 + H_2O_2$$

The polypeptide having superoxide dismutase (hereinafter designated as SOD) activity is useful to scavenge superoxide anion radicals, which are detrimental to cells and tissues. It has been used in the treatment of rheumatic arthritis [Current Therapeutic Res., 20: 62 - 69 (1976)] and as an anti-inflammatory agent for the skin [PCT. No. 62-501072].

EP-A-225,130 and EP-A-240,346 describe a liposome composition containing a drug. The drug may be an anti-inflammatory analgesic such as manganese superoxide dismutase or superoxide dismutase-PEG.

WO 87/01387 describes a particular superoxide dismutase of recombinant origin. The discussion of prior art refers to Cu,Zn SOD.

US-A-4,024,247 describes a composition which has superoxide dismutase activity. This composition has a variety of medical uses, such as facilitating tissue repair, treating cirrhosis of the liver, scar tissue or arthritis and inhibiting inflammation.

We have observed that a polypeptide having SOD activity, which is Cu,Zn-superoxide dismutase, when administered per se to animals with hepatopathy induced by drug injection, gave no decrease in the level of serum GOT and GPT, a marker of hepatopathy.

We have, however, found that a liposomal preparation of the polypeptide having SOD activity administered to animals suffering from hepatopathy unexpectedly reduced the level of serum GOT and GPT effectively.

The present invention provides the use of liposomes comprising a polypeptide having superoxide dismutase activity in the preparation of a composition for the treatment of hepatopathy, wherein said polypeptide is human Cu,Zn-superoxide dismutase or a mutein thereof wherein cysteine at position 111 has been replaced by serine.

The composition defined above may be prepared by dispersing an aqueous solution of the polypeptide in a phospholipid to form liposomes.

As the polypeptide an extracted and purified polypeptide of natural origin, a polypeptide prepared by chemical synthesis or genetic engineering means, or a mutein [Glossary of Genetics and Cytogenetics, 4th Ed., p. 381 (1976)] which is a partially modified peptide with SOD activity provided by chemical synthesis or genetic engineering, is preferably used. The polypeptide contains copper and zinc, is a dimer and has a M.W. of approximately 32,000. It is widely distributed in the placenta, liver or blood of humans.

The human Cu,Zn-SOD polypeptide is constituted by at least 153 amino acids, and can be obtained by the following procedures.

RNA is isolated from human liver tissue, further poly(A) + RNA is isolated, then c-DNA is synthesized therefrom by an ordinary genetic engineering technique. The c-DNA is inserted in a plasmid to prepare an expression vector, which is then transformed into a microorganism such as E. coli or yeast. These microorganisms are cultured to produce a diner polypeptide having SOD activity. These genetic engineering techniques are disclosed in Japanese Patent Unexamined Publication No. 60-137286, ibid. No. 61-111690, ibid. No. 61-139390, ibid. No. 62-115277 and ibid. No. 62-130689.

The polypeptide may also be a mutein polypeptide in which cysteine at position III has been replaced by serine maintaining the SOD activity. For example the expression vector obtained hereinbefore is subjected to site-specific recombination. Namely a closed circular plasmid vector containing DNA corresponding to a polypeptide having natural human type SOD activity is treated with restriction enzyme Bam HI in the presence of ethidium bromide under dark conditions to prepare open circular DNA, which is treated with exonuclease II. Then the treated DNA is annealed with another single-strand DNA which contains a base sequence coding an amino acid to be modified, and the mixture is treated with DNA polymerase and $T_4$ ligase in the presence of dNTP to bind synthetic DNA into a plasmid vector [Experimental Manipulation of Gene Expression, 291-303 (1983), Academic Press. ]. The thus obtained vector in which the expected specific site of the DNA sequence is modified is tranferred by a common genetic engineering method into microorganisms to express the dimer polypeptide, which is isolated by a generally known method. These genetic engineering methods are disclosed in Japanese Patent Unexamined Publication No. 62-130684. In a mutein polypeptide having natural human type SOD activity, i.e. a dimer Cu,Zn-SOD consisting of 153 amino acids with N-terminal alanine at position-1, cysteine at position-6 and/or at position-111, the cystein at position 111 is converted to serine and the N-terminal is hydrogen or an acetylated group. The cysteine at position -6 is cysteine per se or is converted to a neutral amino acid such as serine, alanine or threonine.

These polypeptides can also be prepared in the form of a non-toxic soluble salt.

A liposome is a small vesicle consisting of lipid bilayer containing a water phase in an inner crust.

A liposomal preparation which contains the polypeptide having SOD activity can be produced by known method, for example that disclosed in Biochim. Biophys. Acta, 135 : 624 - 638 (1967), ibid., 443 : 629 - 643 (1976), ibid., 601 : 559(1980), ibid., 542 : 137(1978), ibid ., 649 : 129(1981), ibid., 394 : 483(1975), ibid., 646 : 1(1 981), ibid., 728 : 339(1983), ibid., 770 : 109(1984), ibid., 817 : 193(1985), U.S. Patent 4,235,871, Biochemistry, 8 : 34 4(1969), Proc. Natl. Acad. Sci. U.S.A., 76 : 145(1979), ibid ., 75 : 4194(1978), Ann. Rev. Biophys. Bioeng., 9 : 467(198 0) or J. Am. Chem. Soc., 106 : 1897(1984).

Many kind of typesand forms of liposomes, for example a small-unilamellar vehicle (SUV), large-unilamellar vehicle (LUV), oligolamellar vehicle (REV), multilamellar vehicle (MLV) and stable plurilamellar vehicle (SPLV), can be used.

The composition of the liposome itself is substantially phospholipid, stabilizing agent and electric charging agent. Examples are natural or synthetic phospholipids, preferably natural phosphatidylcholine such as egg-lecithin and soybean lecithin or their hydrogenation products, saturated synthetic lecithin such as dimyristoyl-phosphatidylcholine, dipalmitoyl phosphatidylcholine and distearoyl phosphatidylcholine, or unsaturated synthetic lecithin such as dioleoyl phosphatidylcholine and dilinoleoyl phosphatidylcholine, or a mixture thereof, or another phospholipid such as sphingomyelin, phosphatidylethanolamine, phosphatidylglycerol, phosphatidylserine and ceramide phosphorylethanolamine, a lipid of a stabilizing agent such as cholesterol, a liposome cationic electric charging agent such as cetylamine and stearylamine, and anionic electric charging agent such as diacetylphospahte, phosphatidylserine and phosphatidic acid. In general, the ratios of phospholipid : stabilizing agent : electric charging agent are 100 to 50 weight parts : 0 to 30 weight parts : 0 to 20 weight parts, preferably 75 to 60 : 20 to 15 : 15 to 10 weight parts. Another preferred example is a lipid composition of phospholipid : electric charging agent of 90 to 85 : 10 to 15 weight parts. If required, a lipid stabilizing agent such as tocopherol or $\beta$-carotene can be added.

A lipid composition prepared as above is dissolved in a single or mixed solvent, for example a volatile soluble medium such as diethyl-ether, chloroform, methylene chloride, ethylacetate, petroleum ether or methanol.

The above lipid composition solution is poured into a round flask and the solvent is removed, such as by a rotary evaporator, to form a thin layer of lipid composition on the inner side of the flask. If necessary the operation of adding and removing solvent is repeated and finally the medium should be removed completely in vacuo.

A solution of the polypeptide (0.01 to 100 mg/mℓ) having SOD activity dissolved in distilled water or buffer solution for injection is added to the lipid composition in the flask at a ratio of 5 to 200 weight parts of the solution to one weight part of the lipid composition. The amount of polypeptide can be 0.01 to 0.5 weight parts per one weight part of lipid composition. The mixture is, if required, warmed to from 35 to 60 °C in a water-bath, stirred and suspended to obtain a liposome solution. The suspension is treated with ultrasonication at 10 KC for 1 to 30 minutes to prepare liposomes of uniform size. By ultrasonicaton, liposomes containing the polypeptide having SOD activity mainly consisting of MLV 50 to 550 nm in diameter can be prepared.

The thus prepared liposomes can be isolated by centrifugation at 10,000 to 20,000 G for 15 to 60 minutes. The liposomes can also be treated by ultracentrifugation for concentration or membrane filtration for sterilization. Injectable pharmaceutical preparations can be prepared by suspending the liposomes in a liquid for injection to produce a suspension, or by adding a lyophilization stabilizing agent such as glucose, sucrose, mannitol or sorbitol at a concentration of from 0.5 to 10 % to the liposome solution and lyophilizing to produce a suspension for intramuscular, subcutaneous or intravenous injection before use. The concentration of the polypeptide having SOD activity is preferably 0.01 to 100 mg per ml of composition for one to five times administration in a day with 0.1 to 2 mℓ in one injection. To the liposome preparation can optionally be added an osmotic pressure regulating agent, antiseptics or a pH controlling agent.

Suppositories of the liposome preparation can also be prepared by mixing a liposome with one or more suppository oleaginous bases, for example a fat or oil such as olive oil, peanut oil, sesame oil, soybean oil, rapeseed oil, camellia oil, cacao butter, lard, lanolin and beef tallow, hydrogenated or acetylated derivatives thereof, and emulsified fat and oil with a surface active agent, or furthermore adding one or more aqueous bases such as glycerogelatin and propyleneglycol thereto to prepare a 1 to 2g per unit suppository formulation.

Granules, capsules or a syrup formulation can also be prepared by adding ingredients such as fillers, lubricants, sweeteners, colours or aromas.

The thus prepared liposomes containing the polypeptide having SOD activity are, as illustrated in the following Examples, useful for therapeutic drug compositions for hepathopathy, which can reduce the serum level of GOT and GPT on drug induced hepatic induced hepatic injury.

The following Examples further illustrate the present invention.

EXAMPLE 1

Production of liposomes containing polypeptide having SOD activity:

A chloroform solution containing 7mM L-$\alpha$-dipalmitoyl phosphatidylcholine (hereinafter designated L- $\alpha$-DPPC), 1mM colesterol and 2mM stearylamine was prepared. The solvent was removed in vacuo from a 250ml sample of the above chloroform solution containing the three above lipids in an amount of 2.5 mM. The mixture was then treated in vacuo of a few mm Hg by an oil rotary vacuum pump for 3 hours to remove the solvent further and to prepare a thin layer lipid membrane. A phosphate buffer solution (4mM, pH 7.2, 100mℓ) of a polypeptide having human type Cu,Zn-SOD activity (hereinafter designated SOD-1, 100mg, specific activity 3200 u/mg), wherein cysteine at position-111 was site-specifically replaced by serine, which was obtained by culturing E. Coli DH1 carrying plasmid pδOD14 prepared according to the method disclosed in JP-A-62-130684, was added thereto and heated at 40 °C for 6 minutes to disperse the lipid, and then treated by ultra-sonication.

The thus obtained suspension was allowed to stand for 3 hours at room temperature and thereafter overnight at 15 °C. No encapsulated free SOD-1 was removed by centrifugation at 12000 rpm, at 4 °C for 60 minutes.

The prepared liposomes containing SOD-1(hereinafter designated L-SOD-1) had the following properties.

| | |
|---|---|
| Particle size | : 205 - 550 nm; average diameter 340 - 370nm. |
| Encapsulation ratio of SOD-1 | : 87 % |
| Specific activity of SOD-1 | : 3200 U/mg |
| Recovery of SOD-1 | : 84 % |

The above were measured according to the following method and equations.

Encapsulation ratio (%)

$$= \frac{\text{SOD total activity-SOD supernatant activity}}{\text{SOD total activity}} \times 100$$

The SOD total activity was measured by adding 1 % triton X-100 to a sample and incubating at 37°C for 30 minutes.

The SOD supernatant activity was measured after centrifuging the sample at 14,000 rpm (approx. 20000 G) for 30 minutes at 4 °C.

The SOD activity was assayed accroding to a NBT-method (Anal. Biochem., 44 : 276 - 287 (1971).

$$\text{Specific activity(U/mg)} = \frac{\text{SOD-total activity}}{\text{Total protein (weight)}}$$

The amount of protein was measured according to the method of Lowry et al.

$$\text{Recovery (\%)} = \frac{\text{SOD in liposome}}{\text{Total SOD used}} \times 100$$

EXAMPLE 2

The SOD-1 in example 1 was replaced by Cu,Zn-SOD extracted from human erthrocytes (hereinafter designated as SOD-2) [Development in Biochemistry, Biological and Clinical Aspects of Superoxide and Superoxide Dismutase 11B : 348 (1980)] to obtain liposomes containing SOD-2 (hereinafter designated L-SOD-2).

EXPERIMENT 1

The effects of L-SOD-1 and L-SOD-2 on drug-induced hepatitis using acetaminophen (hereinafter designated as APAP) were determined as follows:

Male Sprague-Dawley rats were divided into 5 groups each of which consisted of 5 to 8 rats. Rats had been administered intraperitoneally at 25 mg/kg body weight with 3-methylcholanthren (hereinafter designated 3-MC) previously 18 hours before starting treatment.

Each group was separated as follows.

Group I :    Control, non treated group.

Group II :    APAP dissolved in distilled water for injection was administered intraperitoneally at 750 mg/kg body weight.

Group III :    APAP dissolved in distilled water for injection was administered intraperitoneally at 750 mg/kg body weight, and simultaneously L-SOD-1 suspended in distilled water for injection was administered intraperitoneally at 16 mg (weight of SOD-1)/kg body weight: After 12 hours the same amount of L-SOD-1 (16 mg equivalent SOD-1) was again administered intraperitoneally.

Group IV:    APAP dissolved in distilled water for injection was administered intraperitoneally at 750 mg/kg body weight, and simultaneously L-SOD-2 suspended in distilled water for injection was administered intraperitoneally at 16 mg (weight of SOD-2)/kg body weight. After 12 hours the same amount of L-SOD-2 (16 mg equivalent SOD-2) was again administered intraperitoneally.

Group V: APAP dissolved in distilled water for injection was administered intraperitoneally at 750 mg/kg body weight, and simultaneously non-liposome (free) SOD-1 dissolved in distilled water for injection was administered intraperitoneally at 16 mg/kg body weight. After 12 hours the same amount of free SOD-1 (16 mg/kg) was again administered intraperitoneally.

All the animals were anesthetized with ether after 24 hours from the APAP administration, and blood was collected from the abdominal aorta. The animals were then autopsied. The livers were observed macroscopically and their weight measured

The extent of the liver cell damage was assessed by assaying the activities of GOT using Merck Auto GOT (Trade name, Cica-Merck Co.), GPT using Merck Auto GPT (Trade name, Cica-Merck Co.) and LDH (lactate dehydrogenase) using Merck Auto LDH (Trade name, Cica-Merck Co.) in serum. [J. Clin. Chem. Clin. Biochem., 10 : 182 (1972), Methods of Enzymatic Analysis, 3rd Ed. Ed.H.U. Bergmeyer, Weinheim Verlag Chemie (1983)] .

The results are shown in Table 1:

### Table 1

| Group | I | II | III | IV | V |
|---|---|---|---|---|---|
| No.of animals | | | | | |
| at start | 5 | 8 | 8 | 8 | 8 |
| at biopsy | 5 | 3 | 6 | 6 | 3 |
| Mortality(%) | 0 | 63 | 25 | 25 | 63 |
| Liver weight (g/100g) | 3.8[1] ±0.6 | 7.2 ±1.0 | 3.6* ± 0.3 | 3.7 * ±0.4 | 6.8 ± 0.3 |
| S - GOT (10 U/ℓ) | 18 ±1 | 773 ±337 | 14 * ±2 | 16* ± 3 | 1190 ± 171 |
| S - GPT (U /ℓ) | 46 ±6 | 4867 ±4159 | 54 * ±6 | 57* ± 5 | 5000 ± 436 |
| LDH ( 10 U/ ℓ) | 239 ±27 | 5730 ±2403 | 110* ±40 | 108 * ± 55 | 5600 ± 219 |

1) Mean ± SD

*) significant difference from II Group and V Group results

at P < 0.05

As illustrated above, increased levels of liver weight, serum GOT, GPT and LDH caused by 3-MC and APAP-induced liver cell damage of hepatopathy, were protected significantly by administration of L-SOD-1 and L-SOD-2. On the contrary no improvement of clinical examination on drug-induced hepatopathy was observed when free SOD-1 was administered.

EXPERIMENT 2

The effect of L-SOD-1 and L-SOD-2 against galactosamine (hereinafter designated GAL)-induced liver cell damage (hepatopathy) :

Male wister rats were divided into 5 groups each of which consisted 5 to 8 rats.

Group I :      Control, non treated group

Group II :     GAL dissolved in distilled water for injection was administered intraperitoneally at 400 mg/kg body weight. (positive control).

Group III :    GAL dissolved in distilled water for injection was administered intraperitoneally at 400 mg/kg body weight, and simultaneously L-SOD-1 suspended in distilled water for injection was administered intraperioneally at 16 mg (weight of SOD-1)/kg body weight.  After 12 hours the same amount of L-SOD-1 (16 mg equivalent SOD-1) was again administered intraperitoneally.

Group IV:      GAL dissolved in distilled water for injection was administered intraperitoneally at 400 mg/kg body weight, and simultaneously L-SOD-2 suspended in distilled water for injection was administered intraperitoneally at 16 mg (weight of SOD-2)/kg body weight. After 12 hours the same amount of L-SOD-2 (16 mg equivalent SOD-2) was again administered intraperitoneally.

Group V:       GAL dissolved in distilled water for injection was administered intraperitoneally at 400 mg/kg body weight, and simultaneously non-liposomal (free) SOD-1 dissolved in distilled water for injection was administered intraperitoneally at 16 mg/kg body weight dissolved in distilled water for injection. After 12 hours the same amount of free L-SOD-1 (16 mg/kg) was again administered intraperitoneally.

All the animals were sacrificed by ether anesthesia as in experiment 1, and the weight of liver and the GOT and GPT activities were measured.

The results are shown in Table 2:

Table 2

| Group | I | II | III | IV | V |
|---|---|---|---|---|---|
| No.of animals | | | | | |
| at start | 5 | 8 | 7 | 7 | 5 |
| at biopsy | 5 | 8 | 7 | 7 | 5 |
| Liver weight (g/100g) | 2.4[1] ±0.1 | 2.8 ±0.2 | 2.8 ±0.1 | 2.9 ±0.2 | 3.0 ±0.2 |
| S - GOT (10 U/ ℓ ) | 14 ± 3 | 363 ±271 | 195 ±61 | 197 ±52 | 379 ±274 |
| S - GPT ( U/ ℓ ) | 5 ± 1 | 437 ±290 | 242 ±98 | 243 ±99 | 459 ±318 |

1) Mean  ±  SD

As illustrated above, increased levels of serum GOT and GPT caused by GAL-induced liver cell damage were protected by administration of L-SOD-1 and L-SOD-2. On the contrary no improvement was observed when free SOD-1 was administered.

EXAMPLE 3

Production of liposomes containing polypeptide having SOD activity and denatured polypeptide:

A chloroform solution containing 7 mM L- $\alpha$-DPPC 7 1 mM cholesterol and 2 mM stearylamine was prepared. The solvent was removed in vacuo from a 250ml sample of the above chloroform solution containing the three above lipids in an amount of 2.5 mM. The mixture was then treated in vacuo of a few mm Hg by an oil rotary vacuum pump for 3 hours to remove the solvent further and to prepare a thin layer lipid membrane, which was heated at 40°C for 6 minutes and treated by ultrasonication to obtain liposomes (hereinafter designated L). The thus obtained L has particle size mainly 80 - 370 nm, average approx. 200 - 250 nm in diameter.

A phosphate buffer solution (4 mM, pH 7.2, 100mℓ) of SOD-1 (100mg, specific activity 3200 U/mg), which was obtained by culturing E. coli DH1 carring plasmid pSOD 14 prepared according to the method disclosed in JP-A-62-130684 was added to another lipid thin membrane and heated at 40°C for 6 minutes to disperse the lipid, and then treated by ultrasonication.

The thus obtained suspension was allowed to stand for 3 hours at room temperature and thereafter overnight at 15 °C. No encapsulated free residual SOD-1 was removed by centrifugation at 12000 rpm for 60 minutes at 4°C.

The thus prepared L-SOD-1 had the following properties.

Particle size : 205 - 550 nm; average diameter 340 - 370 nm.

Encapsulation ratio of SOD-1

: 87 %

Specific activity of SOD-1

: 3200 U/mg.

Recovery of SOD-1

: 84 %

The above were measured by the same methods as in Example 1.

EXAMPLE 4

The SOD-1 in Example 3 was replaced by denatured SOD-1 (hereinafter designated SODden) which was prepared by treating in an autoclave to prepare liposomes containing SODden (hereinafter designatd L-SODden).

EXPERIMENT 3

The effect of L-SOD-1 on APAP induced hepatitis:

Male Sprague-Dawley rats were divided into 5 groups each of which consisted of 5 or 6 animals.

Rats had been administered intraperitoneally at 25 mg/kg body weight with 3-MC previously 18 hours before starting treatment.

Each group was separated as follows.

Group I : Control, non treated group.

Group II : APAP dissolved in phosphate buffer was administered intraperitoneally at 500 mg/kg body weight.

Group III : APAP dissolved in phosphate buffer was administered intraperitoneally at 500 mg/kg body weight, and simultaneously L-SOD-1 suspended in phosphate buffer was administered intraperioneally at 24 mg (weight of SOD-1)/kg body weight.

Group IV : APAP dissolved in phophate buffer was administered intraperitoneally at 500 mg/kg body weight, and simultaneously L suspended in phosphate buffer was administered intraperitoneally at 168 mg/kg body weight.

Group V : APAP dissolved in phosphate buffer was administered intraperitoneally at 500 mg/kg body weight, and simultaneously free SOD-1 dissolved in phophate buffer was administered intraperitoneally at 24 mg (weight of SOD-1)/kg body weight.

Group VI: APAP dissolved in phosphate buffer was administered intraperitoneally at 500 mg/kg body-weight, and simultaneously L-SODden 24 mg as weight of denatured SOD-1/kg suspended in phosphate buffer was administered intraperitoneally.

All the animals were anesthesized with ether after 6 hours from the APAP administration, and blood was collected from the abdominal aorta. The animals were then autopsied. The liver was observed macroscopically and its weight was measured.

The extent of liver cell damage was assessed by assaying the activities of GOT (Merck Auto GOT, Tradename Cica-Merck Co.), GPT (Merck Auto GPT, Tradename, Cica-Merck Co.) and ALP [J. Clin. Chem. Biochem., 10 : 182 (1972), Methods of Enzymatic Analysis, 3rd Ed., ED. H. U. Bergmeyer, Weinheim, Verlag Chemie (1983)].

The results are shown in Table 3:

Table 3

| | I | II | III | IV | V | VI |
|---|---|---|---|---|---|---|
| No. of animals | 5 | 5 | 5 | 6 | 5 | 5 |
| S - GOT (1OU/l) | 23.7[1)] ±3.2 | 1092.8 ±523.5 | 16.2* ±3.5 | 1002.2 ±673.5 | 1175.5 ±573.5 | 599.6 ±199.4 |
| S - GPT (U/l) | 26 ±7 | 1939 ±1168 | 24* ± 5 | 2248 ±1866 | 1818 ±1299 | 902 ±407 |
| ALP (U/l) | 180 ±46 | 323 ±26 | 148* ±22 | 292 ±25 | 253 ±53 | 326 ±25 |

1) ; Mean ± S.D.
* ; significant difference from Group II, Group IV, Group V and Group VI results at P < 0.05.

As illustrated above, increased levels of serum GOT and GPT caused by 3-MC and APAP-induced liver cell damage of hepatopathy were protected significantly by administering L-SOD-1. However no significant protection was observed by administration of L, free SOD or L-SODden.

**Claims**

1. Use of liposomes comprising a polypeptide having superoxide dismutase activity in the preparation of a composition for the treatment of hepatopathy, wherein said polypeptide is human Cu,Zn-superoxide dismutase or a mutein thereof wherein cysteine at position 111 has been replaced by serine.

2. Use according to claim 1 wherein the polypeptide is a natural polypeptide.

3. Use according to claim 1 wherein the polypeptide is a mutein polypeptide.

4. Use according to any one of claims 1 to 3 wherein the polypeptide is present in an amount of from 0.01 to 100 mg per 1 ml of composition.

**Patentansprüche**

1. Verwendung von Liposomen, die ein Polypeptid mit Superoxid-Dismutase-Aktivität umfassen, bei der Herstellung einer Zubereitung für die Behandlung von Lebererkrankungen, wobei das Polypeptid Human-Cu,Zn-Superoxid-Dismutase oder ein Mutein davon ist, worin der Cystein-Rest in Position 111 durch Serin ersetzt wurde.

2. Verwendung nach Anspruch 1, wobei das Polypeptid ein natürliches Polypeptid ist.

3. Verwendung nach Anspruch 1, wobei das Polypeptid ein Mutein-Polypeptid ist.

4. Verwendung nach einem der Ansprüche 1 bis 3, wobei das Polypeptid in einer Menge von 0,01 bis 100 mg pro 1 ml der Zubereitung zugegen ist.

**Revendications**

1. Utilisation de liposomes comprenant un polypeptide ayant l'activité de superoxyde dismutase dans la préparation d'une composition pour le traitement des maladies du foie, où ledit polypeptide est la Cu,Zn-superoxyde dismutase humaine ou sa mutéine, où la cystéine à la position 111 a été remplacée par la sérine.

2. Utilisation selon la revendication 1, où le polypeptide est un polypeptide naturel.

3. Utilisation selon la revendication 1, où le polypeptide est un polypeptide de mutéine.

4. utilisation selon l'une quelconque des revendications 1 à 3, où le polypeptide est présent en une quantité de 0,01 à 100 mg pour 1 ml de la composition.